# EUROPEAN PATENT APPLICATION

(11) **EP 0 753 576 A2**
(43) Date of publication of application: **15.01.1997**
(21) Application number: 96111306.5
(22) Date of filing: 12.07.1996
(51) Int. Cl.: C12N 9/10, C12P 19/12

(54) **Trehalose phosphorylase and processes for preparation thereof**

(30) Priority: 14.07.1995 JP 201770/95
(71) Applicant: KUREHA CHEMICAL INDUSTRY CO., LTD., Chuo-ku Tokyo (JP)
(72) Inventor: Saitoh, Kohki, Nishiki-machi, Iwaki-shi, Fukushima (JP); Kase, Toshiya, Warabi-shi, Saitama (JP); Takahashi, Eiichi, Kanayama-machi, Iwaki-shi, Fukushima (JP); Takahashi, Eisaku, Tokyo (JP); Konai, Yutaka, Machida-shi, Tokyo (JP)
(74) Representative: Boeters, Hans Dietrich, Dr.

(57) **Abstract**

The present invention provides the processes for the preparation of trehalose phosphorylases by culture of microorganisms, such as Rhizopus or Chaetomium strain, which have not been known the productivity of trehalose phosphorylase.

The obtained trehalose phosphorylase having thermal stability. Therefore, the present invention provides the novel method for producing trehalose and α-D-glucose 1-phosphate.

## Description

### Field of the Invention

This invention relates to processes for preparation of trehalose phosphorylases and uses thereof. Particularly, this invention relates to novel thermally stable trehalose phosphorylases, and processes for preparation and uses thereof.

### Background of the Invention

Heretofore, a trehalose phosphorylase derived from *Euglena gracilis* has been reported as EC 2.4.1.64 in Enzyme Nomenclature 1992 (Academic Press). Also, an enzyme derived from *Flammulina velutipes* which produces trehalose from α-glucose 1-phosphate and glucose has been reported as a trehalose phosphorylase [FEMS Microbiology Letters, *55*, 147-150 (1988)].

However, the above mentioned trehalose phosphorylase derived from *Flammulina velutipes* has not sufficiently been purified. One reason of this deficit is presumed to be caused by its low stability. Therefore, no sufficient enzymic properties thereof have been disclosed, for example, pH or temperature stability, optimal reaction temperature or molecular weight. Furthermore, no sufficient datum on the optimal reaction pH or substrate specificity has been reported. In addition, its productivity of trehalose from α-glucose 1-phosphate and glucose was unsatisfactory.

The trehalose phosphorylase disclosed as EC 2.4.1.64 in Enzyme Nomenclature 1992 (Academic Press) is a trehalose phosphorylase which produces trehalose from β-D-glucose 1-phosphate and D-glucose.

The inventors have been investigating to find a novel thermally stable trehalose phosphorylase which produces trehalose from α-D-glucose 1-phosphate and D-glucose and found an enzyme which produces trehalose from α-D-glucose 1-phosphate and D-glucose and having quite different substrate specificity from that of above mentioned known enzymes.

That is, the inventors have been eagerly investigated to find a heretofore unknown new thermally stable trehalose phosphorylase producing microorganism which produces trehalose from α-D-glucose 1-phosphate and D-glucose and found that culture of microorganisms of *Acremonium*, *Byssochlamys*, *Cercospora*, *Chaetomium*, *Glomerella*, *Humicola*, *Myceliophthora*, *Rhizomucor*, *Rhizopus*, *Rosellinia*, *Sclerotinia*, *Sporidiobolus*, *Sterigmatomyces*, *Thermoascus*, *Thielavia* and *Tyromyces* genera produce trehalose phosphorylases, and found that the trehalose phosphorylases obtained from the above mentioned cultured microorganisms are thermally stable and accomplished the present invention.

The trehalose phosphorylases of the present invention hereinbelow described can produce trehalose from α-D-glucose 1-phosphate and D-glucose.

### Summary of the Invention

One object of the present invention is to provide processes for the preparation of trehalose phosphorylases by culture of microorganisms which have not been known the productivity of trehalose phosphorylase.

The other object of the present invention is to provide two novel thermally stable trehalose phosphorylases which maintain the activity at higher temperatures than those of conventional microorganisms.

One further object of the present invention is to provide processes for the production of trehalose or α-D-glucose 1-phosphate by the trehalose phosphorylases produced by trehalose phosphorylase producing microorganisms or novel thermally stable trehalose phosphorylases.

Therefore, the object of the present invention is to provide processes for the production of trehalose phosphorylases by culture of microorganisms which have not been known the productivity of trehalose phosphorylase. Practically, the object of the present invention is to provide processes for the production of trehalose phosphorylases by culture of microorganisms having trehalose phosphorylase productivity and belonging to *Acremonium*, *Byssochlamys*, *Cercospora*, *Chaetomium*, *Glomerella*, *Humicola*, *Myceliophthora*, *Rhizomucor*, *Rhizopus*, *Rosellinia*, *Sclerotinia*, *Sporidiobolus*, *Sterigatomyces*, *Thermoascus*, *Thielavia* and *Tyromyces* genera, and harvesting trehalose phosphorylases from cultured mixture.

The other object of the present invention is to provide novel thermally stable trehalose phosphorylases which exert their activity at higher temperatures than those of known trehalose phosphorylases.

The other further object of the present invention is to provide processes for the production of a trehalose phosphorylases by culture of microorganisms belonging to *Rhizopus* and *Chaetomium* genera and harvesting the novel trehalose phosphorylases from the cultured mixture.

One further object of the present invention is to provide processes for the production of trehalose from α-D-glucose 1-phosphate and D-glucose by the trehalose phosphorylases produced by above mentioned trehalose phosphorylases producing microorganisms and/or novel thermally stable trehalose phosphorylases.

Still another object of the present invention is to provide a process for the production of α-D-glucose 1-phosphate from trehalose with the novel trehalose phosphorylases produced by above mentioned trehalose phosphorylase producing microorganisms and/or novel thermally stable trehalose phosphorylases.

### Brief Description of Drawings

Fig. 1 shows the optimal pH range of trehalose phosphorylase C of the present invention.

Fig. 2 shows the optimal pH range of trehalose phosphorylase R of the present invention.

Fig. 3 shows the stable pH range of trehalose phosphorylase C of the present invention.

Fig. 4 shows the stable pH range of trehalose phosphorylase R of the present invention.

Fig. 5 shows the optimal temperature ranges of trehalose phosphorylase C and trehalose phosphorylase R of the present invention.

Fig. 6 shows the stable temperature ranges of trehalose phosphorylase C and trehalose phosphorylase R of the present invention.

### Description of the Invention and Preferred Embodiments

Any strain which belongs to *Acremonium*, *Byssochlamys*, *Cercospora, Chaetomium*, *Glomerella*, *Humicola*, *Myceliophthora*, *Rhizomucor*, *Rhizopus*, *Rosellinia*, *Sclerotinia*, *Sporidiobolus*, *Sterigmatomyces*, *Thermoascus*, *Thielavia* and *Tyromyces* genera and has productivity of trehalose phosphorylase can be used including variants thereof. Improved strains prepared by integrating the trehalose phosphorylase cDNA derived from these trehalose phosphorylase producing microorganisms in an expression vector of the other microorganisms to make trehalose phosphorylase expressible in microorganisms of the other species can also be used.

Practical examples of trehalose phosphorylase producing microorganisms such as *Acremonium alabamense* IFO 32241, *Byssochlamys nivea* IFO 30569, *Cercospora beticola* IFO 7398, *Chaetomium thermophilum* IFO 9679, *Glomerella cingulata* IFO 7478, *Humicola grisea* IFO 9854, *Myceliophthora thermophila* IFO 31843, *Rhizomucor pusillus* IFO 4579, *Rhizomucor miehei* IFO 9740, *Rhizopus chinensis* IFO 30499, IFO 4768 and IFO 4737, *Rhizopus azygosporus* IFO 31989, *Rhizopus microsporus* IFO 31988, *Rosellinia necatrix* IFO 5954, *Sclerotinia sclerotiorum* IFO 4876, *Sporidiobolus johnsonii* IFO 6903, *Sterigmatomyces halophilus* IFO 1488, *Thermoascus aurantiacus* IFO 31693, *Thielavia terrestris* IFO 9732, *Thielavia arenaria* IFO 31060 and *Tyromyces palustris* IFO 30339 can be used.

The trehalose phosphorylase derived from *Rhizopus* genus obtained by the present invention, hereinafter referred as 'trehalose phosphorylase R' has following physicochemical properties.
1. Action: Formation of trehalose from α-D-glucose 1-phosphate and D-glucose, hereinafter referred as 'trehalose synthetic reaction'. Formation of α-D-glucose 1-phosphate and D-glucose by the reaction on trehalose in the presence of an inorganic phosphoric acid.
2. Substrate specificity: Action on trehalose as a disaccharide substrate in a phosphorolytic reaction. Action on α-D-glucose 1-phosphate and D-glucose as a sugar donor and as a sugar receptor, respectively, in a disaccharide synthetic reaction.
3. Optimal reaction pH:
   Trehalose synthetic reaction (45°C)
   pH range exhibiting about 50% of maximum activity:
   pH 4.7-6.7
4. pH stability:
   Trehalose synthetic reaction: pH 3.5-12.5 (30°C, 1 hr.)
5. Optimal reaction temperature:
   Trehalose synthetic reaction (pH 5.5)
   Temperature range at about 80% of maximum activity: 42.5-50°C
6. Thermal stability:
   Trehalose synthetic reaction: stable at 42.5°C for 30 min. (pH 6.5)
7. Molecular weight: 200,000-280,000 dalton (by GPC).

Further, the trehalose phosphorylase derived from *Chaetomium* genus obtained by the present invention, hereinafter referred as 'trehalose phosphorylase C' has the following physicochemical properties.
1. Action: Formation of trehalose from α-D-glucose 1-phosphate and D-glucose, hereinafter referred as 'trehalose synthetic reaction'. Formation of α-D-glucose 1-phosphate and D-glucose by the reaction on trehalose in the presence of an inorganic phosphoric acid.
2. Substrate specificity: Action on trehalose as a disaccharide substrate in a phosphorolytic reaction. Action on α-D-glucose 1-phosphate and D-glucose as a sugar donor and as a sugar receptor, respectively, in a synthetic reaction.
3. Optimal reaction pH:
   Trehalose synthetic reaction (45°C)
   pH range exhibiting about 50% of maximum activity: pH 4.7-6.7
4. pH stability:
   Trehalose synthetic reaction: pH 3.5-12.5 (30°C, 1 hr.)
5. Optimal reaction temperature:
   Trehalose synthetic reaction (pH 5.5)
   Temperature range at about 80% of maximum activity: 45-50°C
6. Thermal stability:
   Trehalose synthetic reaction: stable at 55°C for 30 min. (pH 6.5)
7. Molecular weight: 300,000-400,000 dalton (by GPC).

Trehalose phosphorylases which can produce trehalose from α-D-glucose 1-phosphate and D-glucose, having the above mentioned physicochemical properties have not been known and first reported by the present invention.

Also, the inventors of the present invention discovered that microorganisms belonging to *Rhizopus* and *Chaetomium* genera produce these novel trehalose phosphorylases. These enzymes have not been found in the above mentioned genera of microorganisms. Microorganisms which produce said novel trehalose phosphorylases belong to *Rhizopus* and *Chaetomium* genera and any strain which has productivity of trehalose phosphorylase including variants thereof can be used. In addition, any microorganism which can produce trehalose phosphorylases having above mentioned physicochemical properties can be used.

The novel trehalose phosphorylases of the present invention can also be prepared by integrating a gene encoding for the trehalose phosphorylases having above mentioned physicochemical properties or their modified trehalose phosphorylases in a suitable host, followed by culture of the resultant variant host.

In the present invention, microorganisms which produce the novel trehalose phosphorylases having the above mentioned physicochemical properties and having the same or partially same immunochemical properties can also be used. The trehalose phosphorylase having the same or partially same immunochemical properties means a trehalose phosphorylase which give wholly or partially fused precipitin line with trehalose phosphorylase having physicochemical properties of the present invention by the known Ouchterlony double immunodiffusion method (Immunobiochemical Study Method, p.40, 1986, Pub. by Tokyo Kagaku Dozin Co., Ltd.).

The trehalose phosphorylases produced by the above mentioned novel trehalose phosphorylase producing microorganisms and the processes for the production of trehalose or α-D-glucose 1-phosphate with the novel trehalose phosphorylases having above mentioned physicochemical properties and favorable thermal stability has not been known.

Any synthetic or natural medium composed of carbon and nitrogen sources, inorganic salts, vitamins and the other nutrients can be used for the culture of microorganisms of the present invention. Conventional sources such as glucose, sucrose, lactose, fructose, glycerol, starch and wasted molasses can be used as carbon sources, and trehalose may be used also for inducible strains.

Inorganic nitrogen compounds such as ammonium sulfate, ammonium nitrate, ammonium chloride, diammonium hydrogen-phosphate and urea, and organic nitrogen sources such as yeast extract, meat extract, peptone, casamino acid. corn steep liquor and soy bean meal can be used. In addition, inorganic salts such as potassium, sodium, magnesium, phosphate, and iron salts and a minute amount of metal salts can be used. Various surface active agents may be used as antifoamers.

The culture is aerobically carried out at about 20-70°C, preferably at 25-60°C and at initial pH 4.0-9.0, preferably 5.0-8.0 by liquid shaking culture or aeration-agitation culture in a jar fermenter. Furthermore, stand culture or solid culture may be applied.

Preparation of trehalose phosphorylases from enzyme sources is performed as follows. The enzymes generally exist within cells, thus cultured cells are preferably collected by centrifugation or filtration. The isolation and purification of trehalose phosphorylases from the cells is performed by collection of cells with centrifugation, washing of the collected cells with a buffer, suspension of washed cells in a suitable amount of buffer and followed by disruption of cells. The cell disruption is performed with conventional sonication, Waring Blendor™ (Dynamics Corp. of America, U.S.A.), rotation with glass beads in Dynomill Crusher, or decomposition of cell membrane with an enzyme such as lysozyme or an organic solvent such as toluene. After cell disruption, insoluble matters are separated and removed to give a crude enzyme solution. The crude enzyme solution can be used for the preparation of trehalose or α-D-glucose 1-phosphate as it is, but further isolation and purification may be performed.

Methods for isolation and purification of the crude enzyme solution are performed with those of common isolation and purification procedures for protein, such as salting out with ammonium sulfate or solvent precipitation, adsorption on an ion exchange resin, separation through a dialysis membrane, condensation with an ultrafiltration membrane, adsorption on hydroxyapatite, isolation with a hydrophobic carrier, or affinity chromatography. The resultant purified enzyme or crude enzyme may be used as it is, but immobilized enzyme prepared by known methods such as binding with a carrier, crosslinking, and gel entrapment, micro-encapsulation may also be used. Furthermore, immobilized living microorganisms by entrapment using polyacrylamide, κ-carrageenan, alginic acid, and photo-crosslinking resin prepolymer may be applied as biocatalysts.

The activity of the enzymes is determined by the following procedure. That is, a buffer solution of 57 mM potassium phosphate containing 10.8 g of trehalose, 860 mg of glutathione, 17.2 mg of EDTA·2Na and purified water for the rest to make 100 ml in total, pH 7.0, is prepared. A mixture of 1,400 µl of the buffer solution, 100 µl of 20 mM NADP⁺ aqueous solution, 100 µl of 26 mM MgCl₂ aqueous solution, 100 µl of 1.34 mM glucose 1,6-diphosphate aqueous solution, 100 µl of 31 U/ml phosphoglucomutase aqueous solution, 100 µl of 35 U/ml glucose-6-phosphate dehydrogenase aqueous solution and 100 µl of a test sample enzyme solution is incubated at 50°C and the amount of NADPH formed is determined by the absorbance at 340 nm with the passage of time. The quantity of enzyme which produces one µmole of NADPH in one minute under the above reaction conditions is made one Unit.

For the preparation of trehalose from α-D-glucose 1-phosphate and D-glucose with the trehalose phosphorylase of the present invention, 5 mM to 4 M, preferably 50 mM to 3 M of α-D-glucose 1-phosphate and 5 mM to 4 M, preferably 50 mM to 3 M of D-glucose are caused to react in the presence of the trehalose phosphorylase of the present invention at pH 2-10, preferably at pH 4-9, more preferably at pH 5-8 and at reaction temperature of 10-80°C, preferably 15-60°C, more preferably 20-55°C. 0.01 Unit or over, preferably 0.1-1,000, more preferably 1-100 Unit of the trehalose phosphorylase is used for one gram of substrate D-glucose. There is no upper limit in the amount of enzyme used for the reaction and an optimal amount can be determined in consideration of economic point of view.

For the preparation of trehalose from sucrose and D-glucose with the trehalose phosphorylases of the present invention, 5 mM to 4 M, preferably 50 mM to 3 M of sucrose, 5 mM to 4 M, preferably 50 mM to 3M of D-glucose are caused to react in the presence of the trehalose phosphorylase, sucrose phosphorylase and an inorganic phosphoric acid and/or its salt, at pH 2-10, preferably pH 4-9, more preferably at pH 5-8 and at reaction temperature of 10-80°C, preferably 15-60°C, more preferably 20-55°C. The trehalose phosphorylase is used at 0.01 Unit or over, preferably 0.1-1,000 Unit, more preferably 1-100 Unit for one gram of substrate sucrose. Sucrose phosphorylase is used at 0.01 Unit or over, preferably 0.1-1,000 Unit, more preferably 1-100 Unit for one gram of substrate sucrose. There is no upper limit in the amount of enzymes used and an optimal amount can be determined in consideration of economic point of view.

For the preparation of trehalose from sucrose with the trehalose phosphorylases of the present invention, 5 mM to 4 M, preferably 50 mM to 3 M of sucrose, is caused to react in the presence of trehalose phosphorylases of the present invention, sucrose phosphorylase, glucose isomerase and an inorganic phosphoric acid and/or its salt at pH 2-10, preferably pH 4-9, more preferably pH 5-8 and at reaction temperature of 10-80°C, preferably 15-60°C, more preferably 20-55°C. The trehalose phosphorylase is used at 0.01 Unit or over, preferably 0.1-1,000 Unit, more preferably 1-100 Unit for one gram of substrate sucrose. Sucrose phosphorylase is used at 0.01 Unit or over, preferably 0.1-1,000 Unit, more preferably 1-100 Unit for one gram of substrate sucrose. Glucose isomerase is used at 0.01 Unit or over, preferably 0.1-1,000 Unit, more preferably 1-100 Unit for one gram of substrate sucrose. There is no upper limit in the amount of enzymes used and an optimal amount can be determined in consideration of economic point of view.

Sucrose phosphorylase is a known enzyme and any enzyme which forms α-glucose 1-phosphate and fructose from sucrose and an inorganic phosphoric acid and/or a salt thereof can be used regardless of the origin. Commercial sucrose phosphorylase and cultured microorganisms which produce these enzymes can be used. Practically, sucrose phosphorylases produced by microorganisms such as *Leuconostoc mesenteroides* and *Pseudomonas saccharophila* can be used.

Any glucose isomerase which forms glucose from fructose can be used regardless of the origin. Commercial glucose isomerase and cultured microorganisms which produce the enzyme can be used. Practically, glucose isomerase produced by microorganisms such as *Streptomyces* and *Arthrobacter* can be used.

For the preparation of α-D-glucose 1-phosphate from trehalose with trehalose phosphorylases of the present invention, 5 mM to 3 M, preferably 50 mM to 2 M of trehalose and 5 mM to 3 M, preferably 50 mM to 2 M of an inorganic phosphoric acid and a salt thereof are caused to react in the presence of the trehalose phosphorylases of the present invention at pH 2-10, preferably at pH 4-9, more preferably at pH 5-8 and at reaction temperature of 10-80°C, preferably 15-60°C, more preferably 20-55°C. 0.01 Unit or over, preferably 0.1-1,000 Unit, more preferably 1-100 Unit of the trehalose phosphorylase is used for one gram of substrate trehalose. There is no upper limit in the amount of enzyme used and an optimal amount can be determined in consideration of economic point of view.

Inorganic phosphoric acids and/or salts thereof used in the present invention include common inorganic phosphoric acids and salts thereof such as orthophosphoric acid, sodium phosphate, potassium phosphate, sodium dihydrogenphosphate and potassium dihydrogenphosphate, are preferably used as a phosphate buffer solution.

Trehalose or inorganic phosphoric acids can be quantitatively determined using the enzymes of the present invention. As shown by the Reaction formula 1, α-D-glucose 1-phosphate and glucose are formed from trehalose and an inorganic phosphoric acid in the presence of trehalose phosphorylase prepared according to the present invention.

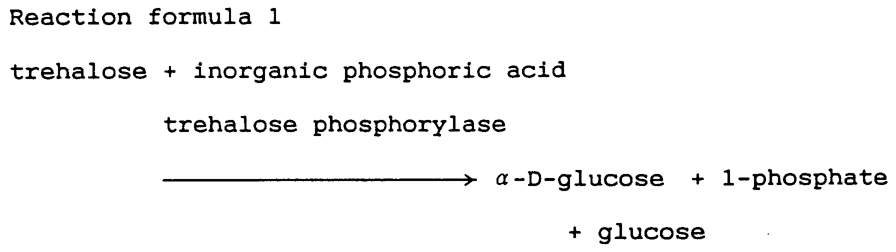

In addition, as shown by Reaction formulae 2 and 3, a combination of phosphoglucomutase [EC 2.7.5.1, Enzyme Handbook, Asakura Pub. Co., Ltd. (1982), similar hereinafter] and D-glucose-6-phosphate dehydrogenase (EC 1.1.1.49) is used. α-D-Glucose 1-phosphate formed by the Reaction formula 1 is converted into D-glucose 6-phosphate by the reaction shown by Reaction formula 2. Then, the resultant D-glucose 6-phosphate is converted into 6-phospho-D-gluconic acid by the reaction shown by Reaction formula 3. The reaction shown by the Reaction formula 3 requires a coenzyme and NAD⁺ and NADP⁺ are converted into NADH and NADPH, respectively. Measurement of formation of NADH or NADPH by the increased absorption rate at 340 nm determines the amount of trehalose or an inorganic phosphoric acid. 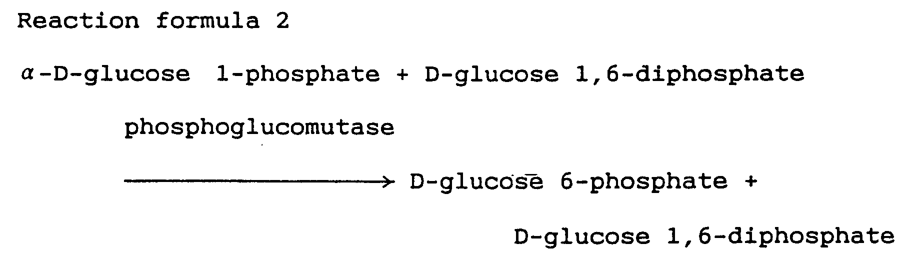 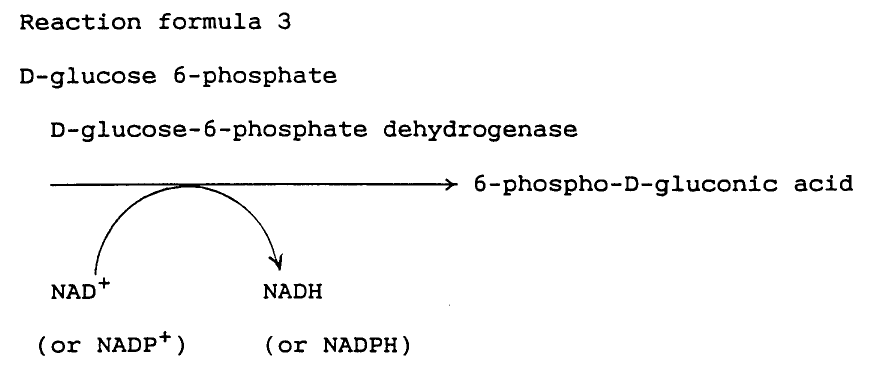 Furthermore, the formed glucose in Reaction formula 1 is measured by a known method using a combination of mutarotase (EC 5.1.3.3), glucose oxidase (EC 1.1.3.4) and peroxidase (EC 1.11.1.7) to determine trehalose or the inorganic phosphoric acid.

According to the present invention, processes for the preparation of trehalose phosphorylases by heretofore unknown microorganisms having productivity of trehalose phosphorylase. In addition, the present invention provides novel trehalose phosphorylases which can be used at higher temperatures than those of conventional trehalose phosphorylases. Further, the present invention provides processes for the preparation of novel trehalose phosphorylases by culture of microorganisms belonging to *Chaetomium* or *Rhizopus* genus. Furthermore, the present invention provides a process for the production of α-D-glucose 1-phosphate and trehalose using these trehalose phosphorylases.

The present invention will be practically explained by the following examples, however, the scope of the present invention is not restricted by these examples.

### [Example 1]

In 1,000 ml of a potato-sucrose medium, 1 g of an yeast extract and 0.5 g of sodium dihydrogenphosphate were added, dissolved and adjusted to pH 5.6. In 300 ml volume Erlenmeyer flasks, 100 ml each of the medium soln. was dividedly poured and pasteurized at 120°C for 15 min. to give a seed culture medium. After pasteurization, *Rhizopus chinensis* IFO 30499 was inoculated and shook at 25°C and 130 rpm for 4 days to give a seed culture. In purified water, 30 g of trehalose, 0.5 g of sodium dihydrogenphosphate, 0.2 g of magnesium sulfate heptahydrate, 1 g of potassium chloride and 20 g of an yeast extract were dissolved, made 1,000 ml, adjusted to pH 6.0 to give a culture medium. In 300 ml volume Erlenmeyer flasks, 100 ml each of the culture medium was dividedly poured, pasteurized in an autoclave. After pasteurization, 5 ml each of the prepared seed culture solution was inoculated and cultured at 28°C and 170 rpm for three days. The cultured mixture was centrifuged to separate cultured solution and cells. The collected cells were washed with a 20 mM Tris buffer, pH 7.5, containing 1 mM EDTA, 1 mM dithiothreitol and 20% glycerol (similar hereinafter). The washed cells were suspended in 20 mM Tris buffer, disrupted with a homogenizer HG-30™ (Hitachi Ltd.) for 2 minutes and centrifuged to give 286 ml of a supernatant of crude enzyme. The crude enzyme solution was charged to a 50 ml of DEAE-Toyopearl™ 650C (TOSOH Corp.) ion exchange column equilibrated with 20 mM Tris buffer, washed with 160 ml of the same buffer, and eluted with 600 ml linear gradient of 20-500mM potassium chloride in 20 mM Tris buffer to give 121 ml of an active enzymatic solution in total. Ammonium sulfate added to the solution and dissolved to make 30% saturation. The obtained solution was charged to a 25 ml of Butyl-Toyopearl™ (TOSOH Corp.) ion exchange column equilibrated with 20 mM Tris buffer containing 30% saturated ammonium sulfate. The column was washed with 70 ml of 20 mM Tris buffer containing 30% saturated ammonium sulfate, eluted with 400 ml linear gradient of 30-0% saturated ammonium sulfate in 20 mM Tris buffer, and collected into 10 ml each fractions. The enzymic activity was found in fraction Nos. 26-39 and 150 ml in total. The active eluate was concentrated with a hollow fiber ultrafiltration apparatus to give 3.6 ml of partially purified enzyme solution. Trehalose phosphorylase activity and total activity of the partially purified enzyme solution was 1.7 unit/ml and 6.1 unit, respectively.

### [Example 2]

One strain selected from those listed in Table 1 was inoculated in YM medium (Defco Co., Ltd.) and cultured to give a seed culture. In 1,000 ml of purified water, 6 g of an yeast extract and 6 g of a malt extract, 10 g of peptone, 10 g of glucose and 20 g of trehalose were dissolved and adjusted to pH 6.2 to give a medium. In 300 ml volume Erlenmeyer flasks, 100 ml each of the medium was poured, pasteurized, inoculated with the seed culture, and cultured according to the culture temperature and period shown in Table 1. Cultured cells were harvested from the resultant cultured mixture and washed with 20 mM Tris buffer containing 1 mM EDTA, 1 mM dithiothreitol and 20% glycerol at pH 7.5 (similar hereinafter). The washed cells were suspended in 20 mM Tris buffer, disrupted with a homogenizer HG-30™ (Hitachi Ltd.) for 2 minutes and centrifuged to give a crude enzyme solution. The trehalose phosphorylase activity and total activity of strains are shown in Table 1.

**[Table 1]**

| Strain | Culture | | Activity | |
|---|---|---|---|---|
| | Temperature (°C) | Period (days) | Activity (Unit/ml) | Total activity (Unit) |
| *Acremonium alabamense* IFO 32241 | 37 | 4 | 0.039 | 2.1 |
| *Byssochlamys nivea* IFO 30569 | 37 | 4 | 0.023 | 3.6 |
| *Humicola grisea* IFO 9854 | 37 | 4 | 0.16 | 3.3 |
| *Myceliophthora thermophila* IFO 31843 | 37 | 4 | 0.007 | 0.2 |
| *Rhizopus chinensis* IFO 4768 | 25 | 7 | 0.024 | 2.0 |
| *Rhizopus azygosporus* IFO 31989 | 30 | 7 | 0.067 | 2.9 |
| *Rhizomucor pusillus* IFO 4579 | 37 | 4 | 0.027 | 0.58 |
| *Rhizomucor miehei* IFO 9740 | 37 | 4 | 0.16 | 0.37 |
| *Rosellinia necatrix* IFO 5954 | 30 | 4 | 0.06 | 0.35 |
| *Sclerotinia sclerotiorum* IFO 4876 | 28 | 4 | 0.035 | 1.8 |
| *Cercospora beticola* IFO 7398 | 28 | 5 | 0.07 | 1.5 |
| *Thermoascus aurantiacus* IFO 31693 | 37 | 4 | 0.03 | 1.8 |
| *Thielavia terrestris* IFO 9732 | 30 | 4 | 0.045 | 1.4 |

### [Example 3]

In 1,000 ml of purified water, 10 g of an yeast extract and 20 g of Bacto-peptone and 20 g of trehalose were dissolved and adjusted to pH 6.0 to give a medium. In 300 ml volume Erlenmeyer flasks, 100 ml each of the medium was poured and pasteurized. In two flasks, *Sporidiobolus johnsonii* IFO 6903 or S*terigmatomyces halophilus* IFO 1488 was inoculated and cultured with shaking at 25°C for two days. The cultured mixture was centrifuged to separate supernatant and cultured cells and the collected cells were washed with 20 mM Tris buffer containing 1 mM EDTA, 1 mM dithiothreitol and 20% glycerol, at pH 7.5, similar hereinafter. The washed cells were suspended in 20 mM Tris buffer, disrupted with a high speed shaking disrupting apparatus (Michael Co., Ltd.) for 15 minutes and centrifuged to separate insoluble matters and to give 20 ml and 34 ml supernatant, respectively. The trehalose phosphorylase activity and total activity of the crude enzyme solutions were 0.46 unit/ml and 9.3 unit for S*poridiobolus johnsonii* IFO 6903 and 0.14 unit/ml and 4.6 unit for *Sterigmatomyces halophilus* IFO 1488, respectively.

### [Example 4]

In YM medium, *Tyromyces palustris* IFO 30339 was seed cultured. In 1,000 ml of purified water, 7.5 g of an yeast extract and 2 g of malt extract, 0.5 g of potassium dihydrogenphosphate, 0.5 g of magnesium sulfate heptahydrate and 40 g of glucose were dissolved and adjusted to pH 5.5. In 300 ml volume Erlenmeyer flasks, 100 ml each of the medium was poured and pasteurized. In two pasteurized flasks, the prepared seed culture was inoculated and cultured at 25°C for 7 days with shaking. After the culture, a crude enzyme solution was prepared in a similar manner with that of Example 1. The trehalose phosphorylase activity and total activity of the crude enzyme solution was 0.16 unit/ml and 7.3 unit, respectively.

### [Example 5]

Strains enumerated in Table 2 were cultured and crude enzyme solutions were prepared. The crude solutions were partially purified to give the partially purified enzyme solutions as shown below. In 1,000 ml of purified water, 6 g of an yeast extract, 6 g of a malt extract, 10 g of peptone, 5 g of glucose and 20 g of trehalose were dissolved, adjusted to pH 6.2 and pasteurized to give a seed culture medium. Strains enumerated in Table 2 were inoculated and cultured to give seed cultures. In 1,000 ml of purified water, 10 g of glucose, 20 g of trehalose, 20 g of an yeast extract, 2 g of potassium dihydrogenphosphate, 0.4 g of dipotassium hydrogenphosphate, and 0.2 g of magnesium sulfate heptahydrate were dissolved and adjusted to pH 6.3. In 300 ml volume Erlenmeyer flasks, 100 ml each of the medium was poured and pasteurized. Then, seed cultures were inoculated and cultured according to culture temperature and period shown in Table 2.

After the culture, crude enzyme solutions were prepared in a similar manner with that in Example 1. The trehalose phosphorylase activity and total activity of the crude enzyme solutions are shown in Table 2. The crude enzyme solutions were partially purified with DEAE-Toyopearl™ (TOSOH Corp.) chromatography and Butyl-Toyopearl™ (TOSOH Corp.) chromatography, and concentrated with ultrafiltration using hollow fiber to give partially purified enzyme solutions. The trehalose phosphorylase activity and total activity of the crude enzyme solutions are shown in Table 2. All activities were determined at 50°C.

**[Table 2]**

| Strain | Culture | | Activity* | |
|---|---|---|---|---|
| | Temperature (°C) | Period (days) | Activity (Unit/ml) | Total activity (Unit) |
| *Acremonium alabamense* IFO 32241 | 35 | 4 | 0.085 | 13 |
| | | | 1.9 | 6.8 |
| *Thermoascus aurantiacus* IFO 31693 | 35 | 6 | 0.032 | 7.7 |
| | | | 0.23 | 4.5 |
| *Rhizopus chinensis* IFO 4737 | 27.5 | 8 | 0.31 | 58 |
| | | | 4.8 | 41 |
| *Rhizopus microsporus* IFO 31989 | 27.5 | 3 | 0.13 | 54 |
| | | | 4.1 | 29 |
| *Thielavia arenaria* IFO 31060 | 35 | 6 | 0.11 | 28 |
| | | | 1.4 | 19 |

| | | | | |
|---|---|---|---|---|
| * Upper column: Crude enzyme solution. Lower column: Partially purified enzyme solution. | | | | |

### [Example 6]

In YM medium (Defco Co., Ltd.), *Glomerella cingulata* IFO 7478 was cultured at 28°C for 4 days with shaking to give a seed culture. In 1,000 ml of purified water, 20 g of an yeast extract, 0.5 g of sodium dihydrogen phosphate, 1 g of potassium chloride, 0.2 g of magnesium sulfate heptahydrate and 30 g of trehalose were dissolved, adjusted to pH 6.3 to give a medium. In the medium, the seed culture was inoculated and cultured at 28°C for 4 days with shaking. After the culture, a crude enzyme solution was prepared in a similar manner with that of Example 1 by collecting cultured cells by centrifugation, washing, cell disruption with a homogenizer HG-30™ (Hitachi Ltd.), and centrifugation. Trehalose phosphorylase activity and total activity of the crude enzyme solution derived from *Glomerella cingulata* IFO 7478 was 0.014 unit/ml and 1.5 unit, respectively.

### [Example 7]

In 1,000 ml of a potato-sucrose medium, 1 g of an yeast extract and 0.5 g of sodium dihydrogenphosphate were added and adjusted to pH 5.6 to give a seed culture medium. In 300 ml volume Erlenmeyer flasks, 100 ml each of the seed culture medium was poured and pasteurized at 120°C for 15 minutes. After pasteurization, *Chaetomium thermophilum* IFO 9679 was inoculated and cultured at 35°C for 6 days with shaking at 150 rpm to give a seed culture. In purified water, 30 g of trehalose, 0.5 g of sodium dihydrogen phosphate, 0.2 g of magnesium sulfate heptahydrate, 1 g of potassium chloride and 20 g of the yeast extract were dissolved, made 1,000 ml adjusted to pH 6.0 to give a culture medium. In 300 ml volume Erlenmeyer flasks, 100 ml each of the culture medium was poured and pasteurized with an autoclave. After pasteurization, 5 ml each of the seed culture was inoculated and cultured at 35°C for 6 days with shaking at 150 rpm.

After the culture, the cultured mixture was centrifuged with a high speed centrifuge to separate into supernatant and cultured cells. The collected cells were washed with 20 mM Tris buffer containing 1 mM EDTA, 1 mM dithiothreitol, and 20% glycerol, at pH 7.5 (similar hereinafter). The washed cells were suspended in 20 mM Tris buffer, disrupted with a homogenizer HG-30™ (Hitachi Ltd.) for 2 minutes and centrifuged to remove insoluble matters and to give 275 ml of a supernatant of crude enzyme solution. Trehalose phosphorylase activity and total activity of the crude enzyme solution were 0.05 unit/ml and 14 unit, respectively.
The crude enzyme solution was charged to a 50 ml of DEAE-Toyopearl™ 650C (TOSOH Corp.) ion exchange column equilibrated with 20 mM Tris buffer, washed with 160 ml of the same buffer, and eluted with linear gradient of 0-500 mM potassium chloride in Tris buffer to-give 215 ml of an active enzyme solution. Ammonium sulfate added to the active solution to give 30% saturation. The obtained solution was charged to a 25 ml of Butyl-Toyopearl™ (TOSOH Corp.) ion exchange column equilibrated with 20 mM Tris buffer containing 30% saturated ammonium sulfate. The column was washed with Tris buffer containing 30% saturated ammonium sulfate, eluted with 400 ml linear gradient of 30-0% saturated ammonium sulfate in 20 mM Tris buffer, and collected into 9.7 ml each fractions. The enzymic activity was found in fraction Nos. 31-46 and 154 ml in total. The active eluate was concentrated with a hollow fiber ultrafiltration apparatus to give 3.6 ml of a partially purified enzyme solution. Trehalose phosphorylase activity and total activity of the partially purified enzyme solution was 3.1 unit/ml and 11 unit, respectively. The active fractions were purified by high performance liquid chromatography (HPLC) using TSK gel™ G3000SW (TOSOH Corp.) column, 7.5 mm ⌀ x 300 mm equilibrated with 20 mM phosphate buffer at a flow rate of 0.5 ml/minute. Detection with UV 280 nm revealed one active peak at elution time of 9.5 minutes. The molecular weight of the peak estimated from comparison of retention time with that of a standard protein sample showed molecular weight of about 380,000.

### [Example 8]

In 1,000 ml of purified water, 6 g of an yeast extract and 6 g of a malt extract, 10 g of peptone, 5 g of glucose and 20 g of trehalose were dissolved, adjusted to pH 6.2 and pasteurized to give a seed culture medium. *Rhizopus azygosporus* IFO 31989 was inoculated and cultured to give a seed culture. In 1,000 ml of purified water, 20 g of the yeast extract, 10 g of glucose, 20 g of trehalose, 4.2 g of potassium dihydrogenphosphate, 0.4 g of dipotassium hydrogenphosphate and 0.2 g of magnesium sulfate heptahydrate were dissolved and adjusted to pH 6.2 to give a culture medium. In 300 ml volume of Erlenmeyer flasks, 100 ml each of the medium was poured and pasteurized. Then, 3 ml each of the seed culture was inoculated and cultured at 27.5°C for 7 days.

After the culture, the cultured mixture was centrifuged with a high speed centrifuge to separate into supernatant and cultured cells. The collected cells were washed with 20 mM Tris buffer containing 1 mM EDTA, 1 mM dithiothreitol and 20% glycerol, at pH 7.5 (similar hereinafter). The washed cells were suspended in 20 mM Tris buffer, disrupted with a homogenizer HG-30™ (Hitachi Ltd.) for 2 minutes and centrifuged to remove insoluble matters and to give 385 ml of supernatant of crude enzyme solution. The trehalose phosphorylase activity and total activity of the crude enzyme solution were 0.56 unit/ml and 217 unit, respectively. Determination of activities were performed in a similar manner to that of the preceding Example except for the reaction was carried out at a temperature of 50°C.

The crude enzyme solution was charged to a 50 ml of DEAE-Toyopearl™ 650C (TOSOH Corp.) ion exchange column equilibrated with 20 mM Tris buffer, washed with 160 ml of the same buffer, and eluted with 600 ml linear gradient of 20-500 mM saturated sulfate of 20 mM Tris buffer to give 130 ml of an active enzyme fraction. Ammonium sulfate added to the active solution to give 30% saturation. The obtained solution was charged to a 25 ml of Butyl-Toyopearl™ (TOSOH Corp.) ion exchange column equilibrated with 20 mM Tris buffer containing 30% saturated ammonium sulfate. The column was washed with Tris buffer containing 30% saturated ammonium sulfate, eluted with 400 ml linear gradient of 30-0% saturated ammonium sulfate in 20 mM Tris buffer to give 103 ml of an active enzyme solution. The active fraction was dialyzed and further purified with a SuperQ Toyopearl™ column to give partially purified enzyme solution having activity of 12 unit/ml and total activity of 113 unit. The enzyme solution was purified by high performance liquid chromatography (HPLC) using TSK gel™ G3000SW (TOSOH Corp.) column, 7.5 mm ⌀ x 300 mm, equilibrated with 20 mM phosphate buffer, at a flow rate of 0.5 ml/min. Detection with UV 280 nm revealed one active peak at elution time of 10.3 minutes. The molecular weight estimated from comparison of retention time with that of standard protein sample showed molecular weight of about 230,000.

### [Example 9]

Substrate specificity, optimal pH, pH stability, optimal temperature, and thermal stability of the enzymes were determined using purified enzymes obtained by Examples 7 and 8.

Quantitative determination of trehalose was carried out as follows.

### Quantitative determination of trehalose:

The concentration of trehalose in the reaction mixture was determined by high performance liquid chromatography (HPLC) using a polyamine column (YMC Pack™, Polyamine 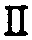 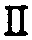 , 4.6 mm ⌀ x 250 mm, YMC Co., Ltd.), an eluent of a mixture of acetonitrile:water = 70:30, flow rate of one ml/min., column temperature of 35°C and a differential refractometer at cell temperature of 35°C. The retention time of trehalose under these HPLC conditions was 15.7 min.

### Trehalose phosphorylase C and trehalose phosphorylase R

### [Substrate specificity]

### Phosphorolytic reaction:

Phosphorolytic reaction of various sugars were performed with the enzymes of the present invention. Trehalose gave α-D-glucose 1-phosphate and D-glucose.

### Disaccharide synthetic reaction:

Disaccharide synthetic reaction from various monosaccharides and α-D-glucose 1-phosphate was performed with the enzymes of the present invention. No reaction was observed in L-glucose, D-galactose, D-mannose, D-xylose, D-fructose, D-sorbitol, D-mannitol or D-fucose except for D-glucose.

### [Optimal pH]

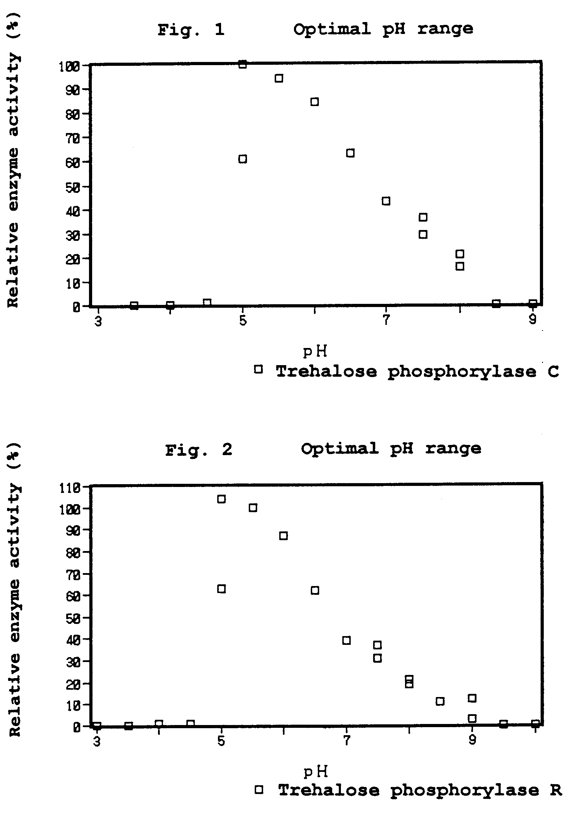

Acetate buffer (pH 3.0-5.5), MES buffer (pH 5.0-7.0), HEPES buffer (pH 7.0-8.0), Tris-HCl buffer (pH 7.5-9.0) and glycine-NaOH buffer (pH 8.5-12.0) were used. The reaction was performed at 45°C for 12 hrs. at these pHs and the formed amount of trehalose was determined by the method shown above to give the enzymic activity. The obtained optimal pH ranges of trehalose phosphorylase C and R are shown in Fig. 1 and 2, respectively.

### [pH stability]

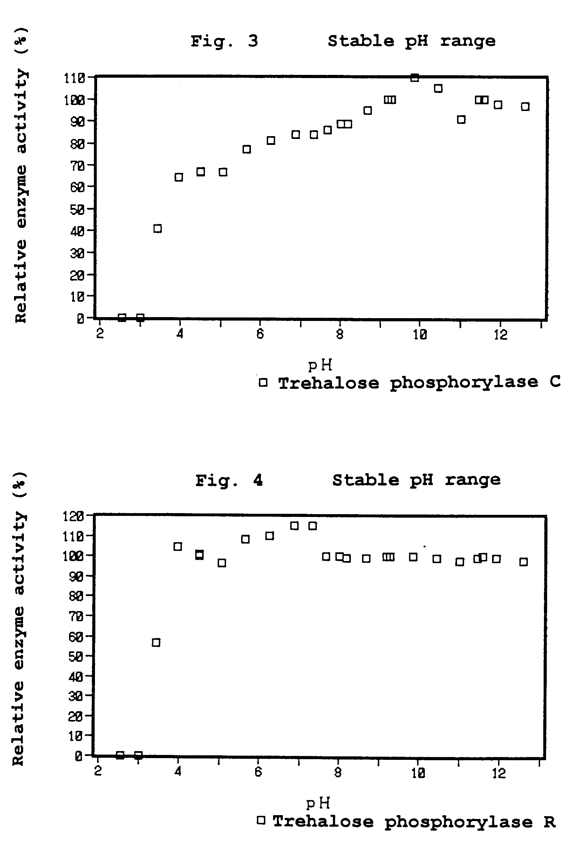

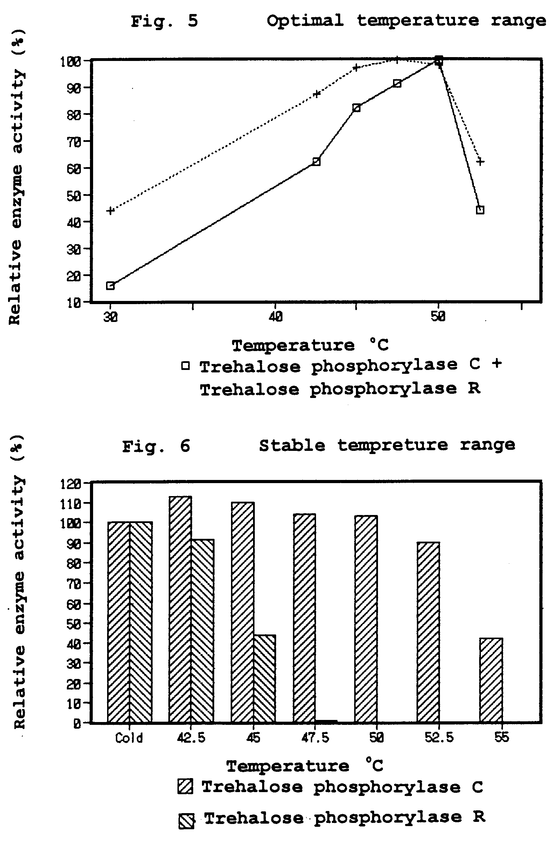

Acetate buffer (pH 3.0-5.5), MES buffer (pH 5.0-7.0), HEPES buffer (pH 7.0-8.0), Tris-HCl buffer (pH 7.5-9.0) and glycine-NaOH buffer (pH 8.5-12.0) were used. Trehalose phosphorylase was added to these buffers and allowed to stand at 30°C for 1 hr. at these pHs, further caused to react at 45°C for 12 hrs., and the quantity of trehalose formed was determined by the HPLC method shown above to give the enzymic activity at various pHs. The obtained pH stability ranges of trehalose phosphorylase C and R are shown in Fig. 3 and 4, respectively.

### [Optimal reaction temperature]

In MES buffer (pH 5.5), the reaction was carried out at selected temperatures between 30 and 55°C for 5 hrs. and the enzyme was inactivated by heating. Then, the amount of trehalose formed was determined by the HPLC method shown above to give the enzymic activity. The obtained optimal temperature ranges of trehalose phosphorylase C and R are shown in Fig. 5.

### [Thermal stability]

In 20 mM phosphate buffer (pH 7.0), trehalose phosphorylase was added and allowed to stand at selected temperatures between 42.5 and 55°C for 30 min. and further at 45°C for 5 hrs. and the enzyme was inactivated by heating. The amount of the formed trehalose was determined by the HPLC method shown above to give the enzymic activity at temperatures tested. The obtained stable temperature ranges of trehalose phosphorylase C and R are shown in Fig. 6.

### [Example 10]

Formation of trehalose from α-D-glucose 1-phosphate and glucose was investigated using partially purified enzymes obtained by Examples 7 and 8. In 100 mM MES buffer. pH 6.5, 100 mM α-D-glucose 1-phosphate, 100 mM glucose and 4 unit/ml of the partially purified enzyme were used for trehalose synthetic reaction at various temperatures for 24 hrs. The results are shown in Table 3.

**[Table 3]**

| Origin of enzyme | Reaction temperature | Formed trehalose (mM) | | | | |
|---|---|---|---|---|---|---|
| | | 30 | 35 | 40 | 45 | 50°C |
| *Chaetomium thermophilum* IFO 9679 | | 1.3 | 11 | 29 | 45 | 55 |
| *Rhizopus azygosporus* IFO 31989 | | 30 | 44 | 60 | 70 | 41 |

### [Example 11]

Formation of α-D-glucose 1-phosphate from trehalose and an inorganic phosphoric acid was investigated using partially purified enzymes obtained by Examples 7 and 8. In 100 mM phosphate buffer, pH 7.0, 100 mM trehalose and 4 unit/ml of the partially purified enzyme were used for α-D-glucose 1-phosphate synthetic reaction at 45°C for 24 hrs. Partially purified trehalose phosphorylases derived from *Chaetomium thermophilus* IFO 9679 and *Rhizopus azygosporus* IFO 31989 formed α-D-glucose 1-phosphate at a rate of 60 mM and 54 mM, respectively. The determination of α-D-glucose 1-phosphate was carried out according to the below mentioned method.

### Quantitative determination of α-D-glucose 1-phosphate:

Fifty µl of the test sample solution was mixed with 120 µl of 0.5 M phosphate buffer (pH 7.0), 150 µl of 1.0 M HEPES buffer (pH 7.0), 50 µl of 14.8 mM NADP⁺ aqueous solution, 50 µl of 26 mM magnesium chloride aqueous solution, 50 µl of 1.34 mM α-D-glucose 1,6-diphosphate aqueous solution, 25 µl of 31 unit/ml aqueous solution of phosphoglucomutase (0.78 unit), 25 µl of 35 unit/ml aqueous solution of glucose-6-phosphate dehydrogenase (0.88 unit) and 980 µl of purified water, and incubated at 30°C for 30 min., then the amount of NADPH formed was determined by absorption at 340 nm to give the amount of α-D-glucose 1-phosphate.

### [Example 12]

Formation of trehalose from α-D-glucose 1-phosphate and glucose was investigated using the partially purified enzyme obtained by Example 5. In 100 mM MES buffer, pH 6.5, 100 mM α-D-glucose 1-phosphate, 100 mM glucose and 4 unit/ml of the partially purified enzyme obtained by Example 5 were used for trehalose synthetic reaction at various temperatures for 24 hrs. The results are shown in Table 4.

**[Table 4]**

| Origin of enzyme | Reaction temperature | Formed trehalose (mM) | | | | |
|---|---|---|---|---|---|---|
| | | 30 | 35 | 40 | 45 | 50°C |
| *Acremonium alabamense* IFO 32241 | | 7.6 | 15 | 22 | 9.2 | 0 |
| *Rhizopus chinensis* IFO 4737 | | 15 | 15 | 18 | 17 | 0 |
| *Rhizopus microsporus* IFO 31988 | | 8.8 | 8.4 | 4.2 | 0 | 0 |
| *Thielavia arenaria* IFO 31060 | | 21 | 31 | 46 | 31 | 0.8 |

### [Example 13]

Trehalose synthetic reaction was carried out using a partially purified trehalose phosphorylase derived from *Chaetomium thermophilum* IFO 9679 obtained by Example 7, and partially purified trehalose phosphorylases derived from *Acremonium alabamense* IFO 32241 and *Thielavia arenaria* IFO 31060 obtained by Example 5. A reaction mixture composed of 330 mM glucose and 330 mM sucrose, 200 mM MES buffer, 20 mM phosphate buffer and each 1 unit/ml of respective trehalose phosphorylase or 1 unit/ml of a commercial sucrose phosphorylase (Sigma Co., Ltd.) was caused to react according to the reaction temperature and pH shown in Table 5. The amount of formed trehalose after 20 and 30 hrs. reaction are shown in Table 5.

**[Table 5]**

| Origin of enzyme | Reaction temperature | pH | Formed trehalose (mM) | |
|---|---|---|---|---|
| | | | 20 | 30 (hrs.) |
| *Chaetomium thermophilum* IFO 9679 | 45 | 6.5 | 55 | 58 |
| *Acremonium alabamense* IFO 32241 | 40 | 6.5 | 23 | 29 |
| *Thielavia arenaria* IFO 31060 | 40 | 6.0 | 48 | 59 |

## Claims

1. A process for preparation of trehalose phosphorylase by culture of trehalose phosphorylase producing strain belonging to one of *Acremonium*, *Byssochlamys*, *Cercospora*, *Chaetomium*, *Glomerella*, *Humicola*, *Myceliophthora*, *Rhizomucor*, *Rhizopus*, *Rosellinia*, *Sclerotinia*, *Sporidiobolus*, *Sterigmatomyces*, *Thermoascus*, *Thielavia* and *Tyromyces* genera in a suitable nutrient medium containing carbon and nitrogen sources and inorganic salts to produce trehalose phosphorylases, followed by recovering trehalose phosphorylases from cultured mixture.

2. The process for preparation of trehalose phosphorylase according to Claim 1, wherein trehalose phosphorylase producing strain is one of *Acremonium alabamense*, *Byssochlamys nivea*, *Cercospora beticola*, *Chaetomium thermophilum*, *Glomerella cingulata*, *Humicola grisea*, *Myceliophthora thermophila*, *Rhizomucor pusillus*, *Rhizomucor miehei*, *Rhizopus chinensis*, *Rhizopus azygosporus*, *Rhizopus microsporus*, *Rosellinia necatrix*, *Sclerotinia sclerotiorum*, *Sporidiobolus johnsonii*, *Sterigmatomyces halophilus*, *Thermoascus aurantiacus*, *Thielavia terrestris*, *Thielavia arenaria* and *Tyromyces palustris* species.

3. The process for preparation of trehalose phosphorylase according to Claim 2, wherein trehalose phosphorylase producing strain is one of *Acremonium alabamense* IFO 32241, *Byssochlamys nivea* IFO 30569, *Cercospora beticola* IFO 7398, *Chaetomium thermophilum* IFO 9679, *Glomerella cingulata* IFO 7478, *Humicola grisea* IFO 9854, *Myceliophthora thermophila* IFO 31843, *Rhizomucor pusillus* IFO 4579, *Rhizomucor miehei* IFO 9740, *Rhizopus chinensis* IFO 30499, *Rhizopus chinensis* IFO 4768, *Rhizopus chinensis* IFO 4737, *Rhizopus azygosporus* IFO 31989, *Rhizopus microsporus* IFO 31988, *Rosellinia necatrix* IFO 5954, *Sclerotinia sclerotiorum* IFO 4876, *Sporidiobolus johnsonii* IFO 6903, *Sterigmatomyces halophilus* IFO 1488, *Thermoascus aurantiacus* IFO 31693, *Thielavia terrestris* IFO 9732, *Thielavia arenaria* IFO 31060 and *Tyromyces palustris* IFO 30339.

4. Trehalose phosphorylase R which forms trehalose and an inorganic phosphoric acid by action on α-D-glucose 1-phosphate and D-glucose, and having the following physicochemical properties:
Optimal pH: pH 5.0-6.0 at 45°C,
pH stability: stable in a range of pH 3.5-12.5 at 30°C for 1 hr.,
Optimal temperature: 42.5-50°C at pH 6.5,
Thermal stability: stable at 42.5°C or lower at pH 5.5 for 30 min.

5. Trehalose phosphorylase R according to Claim 4 produced by a strain of *Rhizopus* genus and forms α-D-glucose 1-phosphate and D-glucose by action on trehalose and an inorganic phosphoric acid or a salt thereof, and trehalose and an inorganic phosphoric acid by action on α-D-glucose 1-phosphate and D-glucose.

6. Trehalose phosphorylase R according to Claim 5 wherein said strain of *Rhizopus* genus is a strain of *Rhizopus azygosporus* species.

7. Trehalose phosphorylase R according to Claim 6 wherein said strain of *Rhizopus azygosporus* species is *Rhizopus azygosporus* IFO 31989.

8. Trehalose phosphorylase R according to Claim 4 wherein trehalose phosphorylase R producing microorganism is a variant of strain belonging to *Rhizopus* genus or a transformant introduced with a gene of trehalose phosphorylase R by gene technology.

9. A process for preparation of trehalose phosphorylase R by culture of trehalose phosphorylase R producing strain belonging to *Rhizopus* genus or a transformant introduced with a gene of trehalose phosphorylase R in a suitable nutrient medium containing carbon and nitrogen sources and inorganic salts to produce trehalose phosphorylase R followed by recovering trehalose phosphorylase R from cultured mixture.

10. Trehalose phosphorylase C which forms trehalose and an inorganic phosphoric acid by action on α-D-glucose 1-phosphate and D-glucose, and having the following physicochemical properties:
Optimal pH: pH 5.0-6.0 at 45°C,
pH stability: stable in a range of pH 3.5-12.5 at 30°C for 1 hr.
Optimal temperature: 45-50°C at pH 6.5,
Thermal stability: stable at 55°C or lower at pH 6.5 for 30 min.

11. Trehalose phosphorylase C according to Claim 10 produced by a strain of *Chaetomium* genus and forms α-D-glucose 1-phosphate and D-glucose by action on trehalose and an inorganic phosphoric acid or a salt thereof, and trehalose and an inorganic phosphoric acid by action on α-D-glucose 1-phosphate and D-glucose.

12. Trehalose phosphorylase C according to Claim 11 wherein said strain of *Chaetomium* genus is a strain of *Chaetomium* *thermophilum* species.

13. Trehalose phosphorylase C according to Claim 12 wherein said strain of *Chaetomium thermophilum* species is a strain of *Chaetomium thermophilum* IFO 9679.

14. Trehalose phosphorylase C according to Claim 10 wherein trehalose phosphorylase C producing microorganism is a variant of strain belonging to *Chaetomium* genus or a transformant introduced with a gene of trehalose phosphorylase C by gene technology.

15. A process for preparation of trehalose phosphorylase C by culture of trehalose phosphorylase C producing strain belonging to *Chaetomium* genus or a transformant introduced with a gene of trehalose phosphorylase C in a suitable nutrient medium containing carbon and nitrogen sources and inorganic salts to produce trehalose phosphorylase C, followed by recovering trehalose phosphorylase C from cultured mixture.

16. A process for preparation of trehalose by causing a reaction of α-D-glucose 1-phosphate and glucose in an aqueous medium to form trehalose in said aqueous medium in the presence of trehalose phosphorylase prepared according to one of Claims 1-3, 9 and 15, followed by recovery thereof.

17. A process for preparation of trehalose by causing a reaction of sucrose, glucose, an inorganic phosphoric acid and/or a salt thereof in the presence of sucrose phosphorylase and trehalose phosphorylase prepared according to one of Claims 1-3, 9 and 15 to form trehalose in an aqueous medium, followed by recovery thereof.

18. A process for preparation of trehalose by causing a reaction of sucrose, an inorganic phosphoric acid and/or a salt thereof in the presence of trehalose phosphorylase prepared according to one of claims 1-3, 9 and 15, sucrose phosphorylase and glucose isomerase to form trehalose in an aqueous medium, followed by recovery thereof.

19. A process for preparation of α-D-glucose 1-phosphate by forming α-D-glucose 1-phosphate from trehalose and an inorganic phosphoric acid and/or a salt thereof in the presence of trehalose phosphorylase prepared according to one of Claims 1-3, 9 and 15 in an aqueous medium, followed by recovery thereof.

20. A process for preparation of trehalose by causing a reaction of α-D-glucose 1-phosphate and glucose in the presence of trehalose phosphorylase according to one of Claims 4-8 and 10-14 to form trehalose in an aqueous medium, followed by recovery thereof.

21. A process for preparation of trehalose by causing a reaction of sucrose, glucose, an inorganic phosphoric acid and/or a salt thereof in the presence of trehalose phosphorylase according to one of claims 4-8 and 10-14 and sucrose phosphorylase to form trehalose in an aqueous medium, followed by recovery thereof.

22. A process for preparation of trehalose by causing a reaction of sucrose, an inorganic phosphoric acid and/or a salt thereof in the presence of trehalose phosphorylase according to one of claims 4-8 and 10-14, sucrose phosphorylase and glucose isomerase to form trehalose in an aqueous medium, followed by recovery thereof.

23. A process for preparation of α-D-glucose 1-phosphate by causing a formation of α-D-glucose 1-phosphate from trehalose, an inorganic phosphoric acid and/or a salt thereof in the presence of an enzyme source composed of trehalose phosphorylase according to one of Claims 4-8 and 10-14, followed by recovery thereof.
